(19) Europäisches Patentamt
European Patent Office
Office européen des brevets

(11) EP 3 976 125 B1

(12) EUROPEAN PATENT SPECIFICATION

(45) Date of publication and mention of the grant of the patent:
**10.04.2024 Bulletin 2024/15**

(21) Application number: **20731245.5**

(22) Date of filing: **29.05.2020**

(51) International Patent Classification (IPC):
***A61L 27/34*** (2006.01) ***A61L 27/50*** (2006.01)
***A61L 27/52*** (2006.01) ***A61L 27/54*** (2006.01)
***A61L 29/08*** (2006.01) ***A61L 29/14*** (2006.01)
***A61L 29/16*** (2006.01) ***A61L 31/10*** (2006.01)
***A61L 31/14*** (2006.01) ***A61L 31/16*** (2006.01)
***C07D 223/10*** (2006.01) ***C09D 175/02*** (2006.01)
***C08G 71/02*** (2006.01) ***C08G 75/04*** (2016.01)
***C07D 233/10*** (2006.01) ***C08L 75/04*** (2006.01)

(52) Cooperative Patent Classification (CPC):
(C-Sets available)
**C08G 71/02; A61L 27/34; A61L 27/50; A61L 27/54; A61L 29/085; A61L 29/14; A61L 29/16; C07D 233/10; C08L 75/04; C09D 175/02;**
A61L 2300/404 (Cont.)

(86) International application number:
**PCT/NL2020/050349**

(87) International publication number:
**WO 2020/242315 (03.12.2020 Gazette 2020/49)**

# (54) MEANS AND METHODS FOR PROVIDING A SUBSTRATE WITH A BIOCIDAL COATING, AND COATED SUBSTRATES OBTAINABLE THEREBY

MITTEL UND VERFAHREN ZUR BEREITSTELLUNG EINES SUBSTRATS MIT EINER BIOZIDEN BESCHICHTUNG UND DADURCH ERHÄLTLICHE BESCHICHTETE SUBSTRATE

MOYENS ET PROCÉDÉS PERMETTANT DE DOTER UN SUBSTRAT D'UN REVÊTEMENT BIOCIDE ET SUBSTRATS REVÊTUS AINSI OBTENUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2019 EP 19177654**

(43) Date of publication of application:
**06.04.2022 Bulletin 2022/14**

(73) Proprietor: **Bioprex Medical B.V.**
**9747 AC Groningen (NL)**

(72) Inventors:
- **LOONTJENS, Jacobus Antonius**
  **9747 AG Groningen (NL)**
- **LI, Rui**
  **9747 AG Groningen (NL)**

(74) Representative: **V.O.**
**P.O. Box 87930**
**2508 DH Den Haag (NL)**

(56) References cited:
**US-A1- 2014 112 994**

- **ROEST STEVEN ET AL: "Charge properties and bacterial contact-killing of hyperbranched polyurea-polyethyleneimine coatings with various degrees of alkylation", APPLIED SURFACE SCIENCE, ELSEVIER, AMSTERDAM, NL, vol. 356, 13 August 2015 (2015-08-13), pages 325-332, XP029310502, ISSN: 0169-4332, DOI: 10.1016/J.APSUSC.2015.08.087 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LARISA M. TIMOFEEVA ET AL: "Secondary and Tertiary Polydiallylammonium Salts: Novel Polymers with High Antimicrobial Activity", BIOMACROMOLECULES, vol. 10, no. 11, 9 November 2009 (2009-11-09), pages 2976-2986, XP055723264, ISSN: 1525-7797, DOI: 10.1021/bm900435v**
- **MINGLUN LI ET AL: "Accurate Determination of Ion Polarizabilities in Aqueous Solutions", JOURNAL OF PHYSICAL CHEMISTRY PART B, vol. 121, no. 26, 21 June 2017 (2017-06-21), pages 6416-6424, XP55648202, US ISSN: 1520-6106, DOI: 10.1021/acs.jpcb.7b04111 cited in the application**
- **J. HERNANDEZ-MONTELONGO ET AL: "Electrostatic immobilization of antimicrobial peptides on polyethylenimine and their antibacterial effect against Staphylococcus epidermidis", COLLOIDS AND SURFACES. B, BIOINTERFACES, vol. 164, 1 April 2018 (2018-04-01), pages 370-378, XP055723500, NL ISSN: 0927-7765, DOI: 10.1016/j.colsurfb.2018.02.002**
- **LARISA TIMOFEEVA ET AL: "Antimicrobial polymers: mechanism of action, factors of activity, and applications", APPLIED MICROBIOLOGY AND BIOTECHNOLOGY, SPRINGER, BERLIN, DE, vol. 89, no. 3, 15 October 2010 (2010-10-15), pages 475-492, XP019874928, ISSN: 1432-0614, DOI: 10.1007/S00253-010-2920-9**
- **LIA A. T. W. ASRI ET AL: "A Shape-Adaptive, Antibacterial-Coating of Immobilized Quaternary-Ammonium Compounds Tethered on Hyperbranched Polyurea and its Mechanism of Action", ADVANCED FUNCTIONAL MATERIALS, vol. 24, no. 3, 1 January 2014 (2014-01-01), pages 346-355, XP055357691, DE ISSN: 1616-301X, DOI: 10.1002/adfm.201301686**


(52) Cooperative Patent Classification (CPC): (Cont.)

C-Sets
**A61L 27/34, C08L 75/02;**
**A61L 27/34, C08L 79/02;**
**A61L 29/085, C08L 75/02;**
**A61L 29/085, C08L 79/02**

## Description

The invention relates to the field of antimicrobial materials, in particular to implantable and other medical devices, exhibiting antimicrobial activity. More specifically, the present invention, which is defined by the present claims, is directed means and methods for providing a substrate, e.g. a catheter or other type of medical device or implant, with an antimicrobial coating comprising surface-immobilized quaternary ammonium compounds, and coated substrates obtainable thereby.

Soluble quaternary ammonium compounds are potent antibacterial compounds and used for more than 70 years. These compounds contain a positively charged nitrogen atom and a hydrophobic tail.

Bacteria have a natural ability to attach themselves to surfaces, both natural and synthetic. Once attached, they often work cooperatively to form biofilms, thin layers of bacterial colonies that can cover the surface of a medical device and introduce the risk of infection. Due to their ability to produce a protective polysaccharide layer they become resistant against the immune system and administered antibiotics. As a result, orthopedic implants, catheters, and even contact lenses can become vehicles for infection. Notably, it has been reported that approximately thirty percent of infections arise less than one month post-implant, another thirty-five percent occur between one month and twelve months post-implant, and the remainder appear more than a year post-implant.

Existing approaches against microbial adhesion use the release of antibiotics or biocidal compounds like silver from a coated surface of an implant. However, the initial burst release of antibiotics followed by a prolonged period minimal release can lead to bacterial resistance and silver ions in body fluids can be toxic, limiting the clinical implementation of these methods. Moreover, releasing system are exhausted within a short period.

Quaternary-ammonium-compounds (QACs) are potent cationic antimicrobials used in everyday consumer products. Surface-immobilized, quaternary-ammonium-compounds create an antimicrobial contact-killing coating. Antimicrobial efficacy of QACs remains preserved and do not contaminate body fluids when QAC-molecules are immobilized on a surface. Such contact-killing coatings have potential in widely-varying applications, including but not limited to surgical equipment and protective apparel in hospitals, medical implants and wound dressings, water purification, food packaging materials and industrial equipment. The use of QACs has been popular since they can be conveniently incorporated in coating systems. QACs are also stable in the human body, poorly metabolized and mainly excreted in nonmetabolized form. QACs can be hemolytic when not immobilized on a surface and environmentally toxic.

Polyethyleneimines (PEIs) are attractive polymers for preparing polycations, as they contain a high density of primary, secondary and tertiary amino groups. Therefore, quaternization of PEI will result in polymeric QACs with a high charge density. The group of Klibanov *et al.* pioneered on alkylation of poly(ethylene imines). Some bactericidal effect was already obtained after *N*-alkylation of the amino groups of PEI with alkyl halides (R= $C_{6-16}$). *N* -alkylation not only resulted in some positive charges on the tethered polymer groups, but also promote hydrophobic interactions. A second alkylation step of N-alkylated $C_{6-16}$-PEIs with methyl iodide resulted in a substantially higher charge density and biocidal efficacy. Due to the small size of the methyl group and the good leaving behaviour of iodide, the degree of quaternization increased substantially, and, as a result, the biocidal activity as well.

Thus, anchored PEIs are typically converted into antimicrobial polyquats by alkylation of the amino groups in a two-step procedure. See for example Behlau et al. (Biomaterials. Dec 2011; 32(34): 8783-8796) disclosing the biocompatibility and antibacterial properties of *N,N*-hexyl, methyl-polyethylenimine (HMPEI) that is covalently attached to the Boston Keratoprosthesis materials. It was found that HMPEI-derivatized materials exert an inhibitory effect on biofilm formation by *Staphylococcus aureus* clinical isolates, as compared to the parent poly(methyl methacrylate) (PMMA) and titanium.

WO2016/043584 in the name of the applicant discloses a coating method involving the "pre-oligomerization" of monomers forming a hyperbranched polyurea to a low molecular weight polyurea prior to contacting the polyurea with the surface to be coated, rather than polymerization of the monomers on the surface. The coating obtained via pre-oligomerization was robust, reproducible and long-lasting, with a homogenous covering of the surface. The coating could be readily provided with antibacterial agents, including *N,N*-alkylated PEI.

Park et al. (Biotechnol. Prog. 2006, 22, 584-589) developed hydrophobic polycationic systems based on QACs that can serve as such a "bactericidal paint". Disclosed is a single-step, general procedure akin to common painting. Glass or polyethylene slides were shortly dipped into organic solutions of certain hydrophobic *N*-alkyl-PEI (where PEI stands for branched 750-kDa polyethylenimine) polycations, followed by solvent evaporation. The *N*-alkyl-PEI was physically absorbed to the surface. The resultant polycation coated slides were able to kill on contact all of the encountered bacterial cells, whether the Gram-positive human pathogen *Staphylococcus aureus* or its Gram-negative brethren *Escherichia coli.* See also US2013/0110237.

It is generally held that antibacterial action of immobilized N-alkylated PEI relies on the fact that N-alkylation introduces a positive charge, as well as a hydrophobic tail. The positive charge is considered to interact with the negative charge of bacteria, whereas the hydrophobic alkyl tail enables penetration into the hydrophobic phospholipid layer of the cytoplasmic membrane. Thus far, this dual effect of alkylation has been considered as indispensable. Moreover, it is believed that the alkyl tail should contain at least six C-atoms, with the biocidal activity increasing with the number of

$CH_2$ moieties, up to about sixteen carbon atoms (Lin et al., Biotechnol. Prog. 2002, 18, 1082-1086; Zhao et al. , Langmuir, 2019, 35 (17), pp 5779-5786; )

However, drawbacks of currently available coatings and coating methods based on (immobilized) N-alkylated PEI include the high costs of alkylhalide reagents and the lengthy two-step procedure in the presence of a base.

The present inventors therefore set out to investigate whether at least some of the above drawbacks could be avoided by a modification of the conventional N-alkylation procedure. Contrary to all expectations, it was surprisingly found that N-alkylation of PEI is not required to convert it to a bactericidal material. Instead, the role of positive charges due to amine protonation was found to be crucial for antibacterial action. More specifically, the surface charge density of immobilized PEI could be increased by the mere addition of a salt comprising a polarizable anion, such as iodide, to values that are high enough to kill bacteria.

Conceivably, full PEI protonation in an aqueous environment cannot be achieved due to the repulsive forces of the vicinal nitrogen atoms, which are too close to all become protonated. Without wishing to be bound by theory, the anions exert a "shielding" effect wherein repulsive forces between positively charged nitrogen atoms are reduced such that the degree of amine protonation is enhanced to a level that is sufficient for bactericidal activity.

Herewith, the present invention shows for the first time that a non-alkylated (i.e. non-quaternized) immobilized polyamine has antimicrobial properties, and that protonation is much more important for QAC-based coatings than alkylation. This finding is highly surprising in view of the prior art described herein above, consistently teaching that the two phenomena associated with PEI alkylation, i.e. a positive charge *and* a hydrophobic alkyl tail, are without any doubt essential for bactericidal action. Furthermore, the group of Klibanov, who is the pioneer of antibacterial quaternary ammonium compounds based on PEI, reported that quaternary amino group density was zero in water when no alkylation step was conducted.[1] In all Klibanovs studies, the alkylation was performed with hexylbromide and methyliodide in the presence of a base to scavenge HBr and HI. Methyliodide is a stronger alkylation agent and gives additional enhancement of the charge density.

Accordingly, in one embodiment the invention provides a method for providing a substrate with an antimicrobial coating, comprising providing a substrate that is coated with a covalently attached polyamine functionalized polymer, wherein the polyamine is non-quaternized and non-alkylated and enhancing the charge density /degree of protonation of the polyamine-functionalized polymer by contacting said polyamine-functionalized polymer in an aqueous environment with a "shielding composition" in the form of an aqueous solution of at least one salt having a high polarizability and which is as defined in the claims. The polyamine is polyethylene imine (PEI).

As used herein, the term "non-quaternized (polyamine)" or 'non-alkylated" refers to (polyamine) compounds that are free of any quaternary ammonium groups, as opposed to QAC's or "polyquats" known in the art. A quaternary ammonium group consists of a central positively charged nitrogen atom with four substituents, especially organic (alkyl and/or aryl) groups. A coating method or coated substrate according to the invention is not disclosed or suggested in the art.

Gibney et al. (Macromol. Biosc. Vol. 12, no. 9, p 1279-1289) relates to the effect of non-alkylated linear and branched PEI on bacteria in solution. Only the MIC (minimum inhibitor concentration) value was measured. MIC is the concentration of a biocide at which bacteria do not grow. It is not an indicator of the bactericidal (killing) effect. Moreover, the MIC value is measured in solution with soluble bacteria, which is very different from polymer-immobilized PEIs according to the present invention. Thus, the mechanism of killing in solution is completely different from that on covalently coated surfaces.

US2014/0112994 relates to a method for forming an antimicrobial metal-containing LbL coating on a medical device. The method comprises alternatively applying, in no particular order, at least one layer of a negatively charged polyionic material having -COOAg groups and at least one layer of a positively charged polyionic material onto a medical device. Exemplary polycationic polymers may comprise primary or secondary amino groups or a salt thereof, including polyethyleneimine (PEI). However, in contrast to the present invention, the LbL coating is not covalently attached to the medical device.

Asri et al. (Adv. Funct. Mater. 2014, 24, 346-355) describe the preparation of a shape-adaptive, contact-killing coating by tethering the double N-alkylated PEI (QAC), onto hyperbranched polyurea coatings.

Yodovin-Farber et al. (J. of Nanomaterials, Vol. 2010, p.1-11) describe the manufacturing of quaternary ammonium polyethyleneimine-(QA-PEI)-based nanoparticles and their antibacterial activity. The QA-PEI was either single or double *N*-alkylated.

Zaltsman et al. (J. Appl. Biomater. Funct Mater 2016: 14(2): e205-e211) relates to preparing similar types of QA-PEI nanoparticles, all involving N-alkylation of PEI. Notably, it is stated therein that a high content of large carbonate ions are likely to mask the quaternary ammonium cations, resulting in reduced antibacterial properties. This is in marked contrast to, and teaching away from, the present invention wherein polarizable anions are used to increase the bactericidal activity of a PEI-based coating.

Wong et al. (Biomaterials 31 (2010); 4079-4087) relates to bactericidal and virucidal ultrathin films using layer-by-layer technology from N-alkylated PEI and polyanions. Nothing is mentioned about conferring bactericidal activity to

non-quaternized PEI using one or more salts having a high ion polarizability.

WO2008/156636 relates to an antimicrobial surface polymer coating capable of killing or deactivating bacterial spores which comprises: (1) hyperbranched polymers having (a) at least one heterocyclic N- halamine terminal group, or (b) at least one quaternary ammonium terminal group, or (c) a mixture comprising at least one each of quaternary ammonium and heterocyclic N-halamine terminal groups; or (2) polyamidoamine dendrimers having at least one heterocyclic N- halamine terminal group, or (3) linear PEI with hydantoin and *N*-alkyl quaternary ammonium groups at each repeat unit. According to WO2008/156636, each repeat unit of PEI carries hydantoin and *N*-alkyl quaternary ammonium groups, which is contrary to the present invention relating to antimicrobial coatings comprising non-alkylated (non-quaternized) PEI.

In one embodiment of the present invention, a method for providing a substrate with an antimicrobial coating comprises providing a substrate that is covalently coated with a polyamine-functionalized polymer, and contacting said polyamine-functionalized polymer with an aqueous salt solution comprising at least one salt having a polarizability $\alpha_{37}$ of at least 4 Å$^3$ determined at 37°C and is as defined in the claims. The polyamine is polyethyleneimine (PEI).

Ion polarizability is defined as the ratio of the induced dipole moment of an ion to the local electric field. Various methods to calculate or predict ion polarizabilities in aqueous solutions have been described in the art. See for example the study of Li et al. (J. Phys. Chem. B, 2017, 121, 6416-6424) who determined the polarizabilities of 32 strong electrolyte salts in aqueous solutions. A novel extrapolation method is presented using refractive index and volume measurements, which method was found to yield results that are in better agreement with the theoretical results obtained from earlier studies. The salt polarizability values thus obtained at 37°C are presented as $\alpha_{37}$ (the last column) in Table 1 of Li *et al.*

Accordingly, the shielding composition for use in a method disclosed herein is an aqueous solution of at least one salt having a polarizability $\alpha_{37}$ of at least 4 Å$^3$ determined at 37°C according to the method of Li et al. (2017). Preferably, the aqueous solution comprises at least one salt having a polarizability $\alpha_{37}$ of at least 4.5 Å$^3$, more preferably at least 5 Å$^3$.

As is known in the art (J. Phys. Chem. B 2017, 121, 6416-6424), the polarizability is related to the refractive index of the solvent and solution according to:

$$R = \frac{4\pi \, N\alpha_{salt}}{3}$$

where R is molar refractivity, N is Avogadro's constant and $\alpha_{salt}$ denotes the sum of polarizabilities of the solvated ions in cgs units or written in another way:

$$\alpha_{salt} = \frac{3R}{4\pi N}$$

$\alpha_{salt}$ can be calculated by measuring the molar refractivity R. R is related to the refractive index and volumes of the liquid before and after addition of salt, according to the Clausius-Mossotti equation:

$$R = \frac{1}{c_{salt}} \left[ \frac{\eta^2 - 1}{\eta^2 + 2} - \frac{\eta_{water^2 - 1} V_{water}}{\eta_{water^2} + 2 \, V_{sol}} \right]$$

Here, $c_{salt}$ is the molar salt concentration, with unit mol/L; $V_{water}$ and $n_{water}$ are the volume and refractive index of pure water, respectively, before the addition of salt; and $V_{sol}$ and $\eta$ are, respectively, the volume and refractive index of the aqueous mixture solution after addition of salt. By measuring the refractive index the sum of polarizabilities of the solvated ions, $\alpha_{salt}$, can be calculated.

Accordingly, in the present invention the aqueous salt solution comprises at least one salt having a polarizability $\alpha_{salt}$ of at least 4 Å$^3$ determined at 37°C, wherein

$$\alpha_{salt} = \frac{3R}{4\pi N}$$

wherein R is molar refractivity, N is Avogadro's constant and $\alpha_{salt}$ denotes the sum of polarizabilities of the solvated ions in cgs units.

In one aspect, the invention provides a method for providing a substrate with an antibacterial coating, comprising

providing a substrate that is covalently coated with a polyamine- functionalized polymer, wherein said polyamine is non-quaternized and non-alkylated, and contacting said polyamine-functionalized polymer with an aqueous salt solution comprising at least one ammonium salt, an alkaline metal salt and/or earth alkaline metal salt of an anion selected from the group consisting of Br-, I-, $ClO_4^-$, $SO_4^{2-}$, $NO_3^-$ and $PO_4^{3-}$.

Preferred alkaline metal salts include $Li^+$, $Na^+$ and $K^+$. Preferred earth alkaline metal salts include $Mg^{2+}$ and $Ca^{2+}$. Noble metal salts like Ag-salts or Au-salts (not part of the invention) are less preferred.

Exemplary aqueous salt solutions are those comprising one or more of NaI, KI, NaBr, KBr, $NaClO_4$, $KClO_4$, $Na_2SO_4$, $K_2SO_4$, $Na_3PO_4$, $K_3PO_4$, $Mg(NO_3)_2$, $Ca(NO_3)_2$, $(NH_4)_2SO_4$, $NH_4NO_3$, $Zn(NO_3)_2$, $NaNO_3$, $NH_4I$, $CaSO_4$, and $Al(NO_3)_3$.

Good results were obtained with aqueous salt solutions comprising NaI, $K_2SO_4$, $Mg(NO_3)_2$, $NH_4I$, $NaNO_3$, and/or $Zn(NO_3)_2$.

The concentration of the salt(s) providing polarizable anions is not critical. Also, the duration of contacting the amine functionalized polymer to the shielding composition can be varied according to needs. Contacting (shielding) periods ranging from 0.5 to 20 hours were found to be effective, but shorter and longer periods are also encompassed. Whereas contacting with the shielding composition is suitably performed at room temperature, any temperature in the range of 0 to 100°C can be used.

It was observed that with increased salt concentration, the treatment period with the shielding composition could be reduced. For example, a high surface charge density and good bactericidal activity was obtained using 1-100 mM NaI.

The efficacy of a shielding salt composition to increase the extent of quaternization of amines, thus the charge density, can be measured using methods known in the art. For example, the Fluorescein method (Tiller et al., PNAS, May 22, 2001, vol. 98, no. 11, 5981-5985) measures the absorption of fluorescein on the positive charged nitrogen by UV spectroscopy. It is performed in water, and measures both alkylated and protonated nitrogen atoms.

As will be appreciated by a person skilled in the art, a method of the invention is suitably used to convert any non-quaternized polyamine-functionalized polymer into a biocidal polymer. In particular, it is a polymer that has heretofore been used to provide QAC's by *N*-alkylation.

Exemplary polyamines that can be used in the present invention to prepare a polyamine-functionalized polymer include the following:

Putrescine 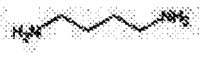

Cadaverine 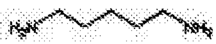

Spermidine 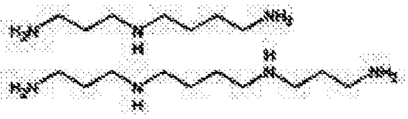

Spermine

Thermospermine 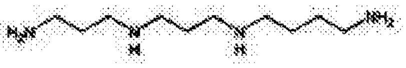

Caldopentamine 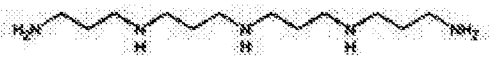

Caldohexamine 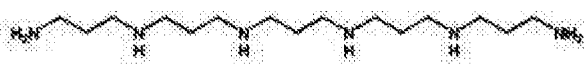

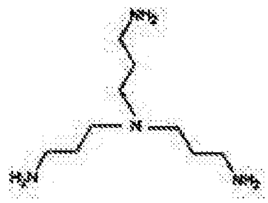

Tris(3-aminopropyl)amine

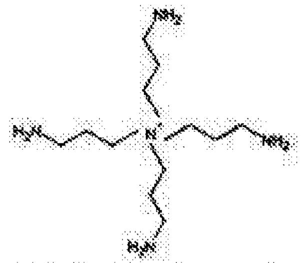

Tetrakis(3-aminopropyl) ammonium

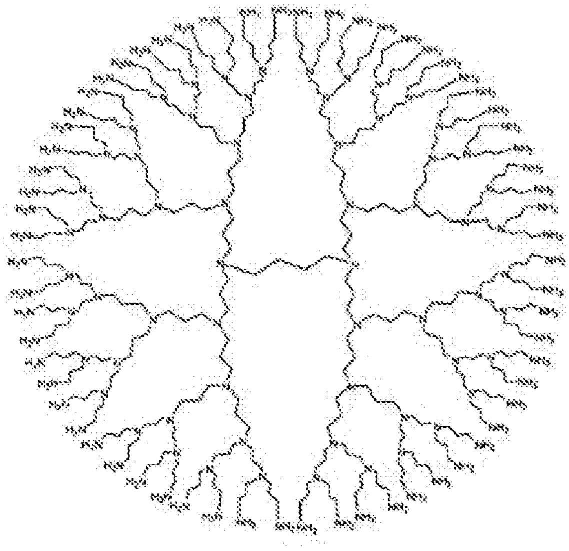

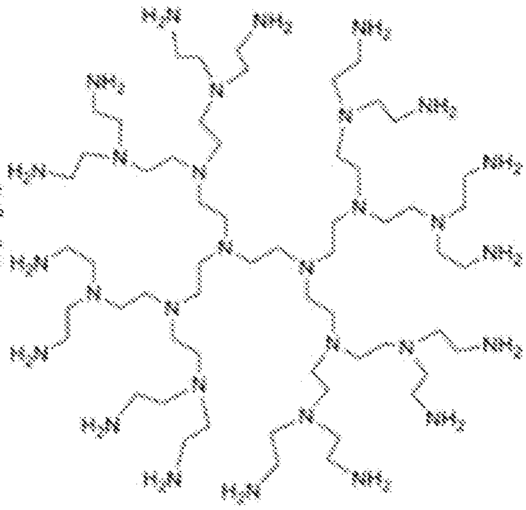

Polypropyleneimine

Polyethyleneinine dendrimer

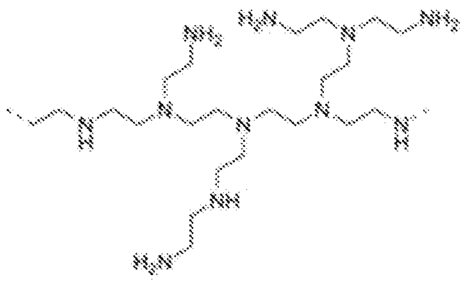

Polyethyleneimine

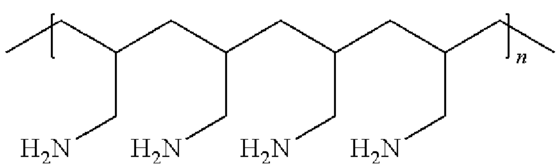

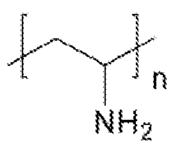

Polyallylamine

Polyvinylamine

Linear

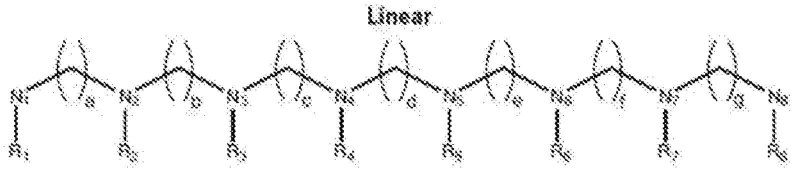

Branched

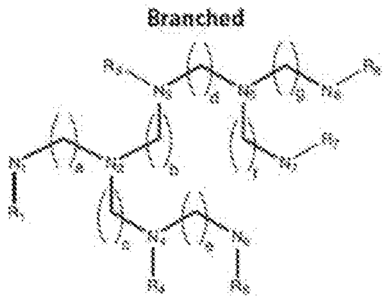

Cyclic

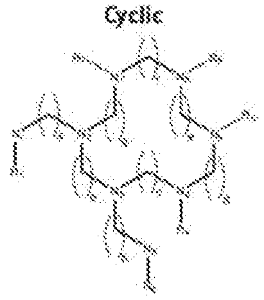

In a preferred embodiment, the polyamine- functionalized polymer comprises or consists of non-quaternized polyethyleneimine (PEI). PEI-based biocides are effective in inhibiting the growth of many microorganisms. As used herein, microorganisms includes single-cell and multi-cell bacteria, fungi, parasites, protozoans, archaea, protests, amoeba, viruses, diatoms, and algae. Microorganisms whose growth may be inhibited by polyethylenimine based coatings of the invention include *Staphylococcus aureus, Staphylococcus epidermidis, Streptococcus faecalis, Bacillus subtilis, Salmonella chloraesius, Salmonella typhosa, Escherichia coli, Mycobacterium tuberculosis, Pseudomonas aeruginosa, Aerobacter aerogenes Saccharomyces cerevisiae, Candida albicans, Aspergillus niger, Aspergillus flares, Aspergillus terreus, Aspergillus verrucaria, Aureobasidium pullulans, Chaetomaum globosum, Penicillum funiculosum, Trichophyton interdigital, Pullularia pullulans, Trichoderm sp. madison P-42,* and *Cephaldascus fragans; Chrysophyta, Oscillatoria bometi, Anabaena cylindrical, Selenastrum gracile, Pleurococcus sp., Gonium sp., Volvox sp., Klebsiella pneumoniae, Pseudomonas fluorescens, Proteus mirabilis, Enterobacteriaceae, Acinetobacter spp., Pseudomonas spp., Candida spp., Candida tropicalis, Streptococcus salivarius, Rothia dentocariosa, Micrococcus luteus, Sarcana lutea, Salmonella typhimurium, Serratia marcescens, Candida utilis, Hansenula anomala, Kluyveromyces marxianus, Listeria monocytogenes, Serratia liquefasciens, Micrococcus lysodeikticus, Alicyclobacillus acidoterrestris, MRSA, Bacillus megaterium, Desulfovibrio sulfuricans, Streptococcus mutans, Cobetia marina, Enterobacter aerogenes, Enterobacter cloacae, Proteus vulgaris, Proteus mirabilis, Lactobacillus plantarum, Halomonas pacifica, Ulva linza,* and *Clostridium difficile.* Preferably, the invention provides a method for providing a substrate with an antimicrobial (e.g. antibacterial) coating, comprising providing a substrate that is coated with non-alkylated PEI, and enhancing the charge density of PEI by contacting the PEI-coated substrate with an aqueous shielding composition as defined in the claims.

For example, the PEI-coated substrate is contacted with an aqueous shielding composition comprising one or more of NaI, KI, NaBr, KBr, $NaClO_4$, $KClO_4$, $Na_2SO_4$, $K_2SO_4$, $Na_3PO_4$, $K_3PO_4$, $Mg(NO_3)_2$, $Ca(NO_3)_2$, $(NH_4)_2SO_4$, $NH_4NO_3$, $NH_4I$, MgSO4, $CaSO_4$, $Zn(NO_3)_2$, $NaNO_3$, and $Al(NO_3)_3$.

A major object of the present invention is to develop an improved process for coating a surface of a substrate to make the surface antimicrobial. Such a surface can be kept substantially and persistently free from microorganisms including bacteria, for example cocci, in a physiologically compatible manner without thereby altering the mechanical properties of the treated materials. The invention therefore is defined by the appended claims and provides a process for coating a surface of a substrate to make the surface antimicrobial, comprising contacting providing a substrate that is coated with a PEI-functionalized polymer, and enhancing the charge density / degree of protonation of the PEI-functionalized polymer by contacting said PEI-functionalized polymer with a shielding composition capable of reducing repulsive forces between positively charged nitrogen atoms of the PEI amines.

The substrate to be coated can be any type of substrate, article, object or material. In modern medicine frequent use is made of exogenous articles in such a way that they come into medium - or long-term contact with tissue or body fluids. Examples are implants, such as pacemakers, stents and prostheses, and also suture materials, drainage hoses and catheters. Such articles may consist, inter alia, of metals, ceramics and/or polymers.

In one embodiment, the substrate is a medical grade material, preferably selected from the group consisting of medical grade polyethylene, polydimethylsiloxane elastomer (PDMS), polyurethane, and polyvinylchloride (PVC).

In another embodiment, the substrate is a metal which is biocompatible with the mammalian body, preferably selected from the group consisting of stainless steel alloys, titanium, titanium-alloy, tantalum and tantalum-alloy.

The PEI-functionalized polymer coating is associated with at least part of the outer surfaces of the substrate in a covalent (fixed) manner. Since leaching out of the biocidal compounds is often undesirable, the polymer coating is

covalently associated with at least part of the surface of the substrate.

Hyperbranched polymers (HBPs) are particularly suitable for specialty coatings. They allow introduction of a wide variety of (bio-active) functionalities, such as biocides, due to the numerous end groups. The anchoring of coatings on surfaces can be achieved either by physical adsorption or by covalent bonding. Although physical adsorption is a common application technique, the coating layers can be removed under harsh application conditions. In contrast, covalently attached coating layers according to the present invention can withstand harsh conditions better. Therefore, in a preferred embodiment, the covalently attached polymer coating comprises a hyperbranched coating. Hyperbranched polymers can be prepared by chain and step growth polymerizations. The most important routes to prepare HBPs are either by polymerizing of $A_2$ and $B_x$ monomers or by using $AB_x$ monomers. A and B represent two different functional groups that are able to react with each other, and x is the number of B groups in a monomer.

In a specific aspect, the invention, which is defined by the claims, provides a method for providing a substrate with an antibacterial coating, comprising providing a substrate that is coated with a PEI-functionalized hyperbranched polyurea, and enhancing the charge density of the amine functionalized coating by contacting the coated substrate with a shielding composition capable of reducing repulsive forces between positively charged nitrogen atoms of said amines.

A PEI-functionalized hyperbranched polyurea can be obtained by using methods known in the art. See for example Asri et al. Adv. Funct. Mater. 2014, 24, 346-355. It may comprise the polymerization of $AB_2$ monomers comprising a secondary amine as A-group and blocked isocyanates as B-groups.

For example, the $AB_2$ monomers are of the general formula

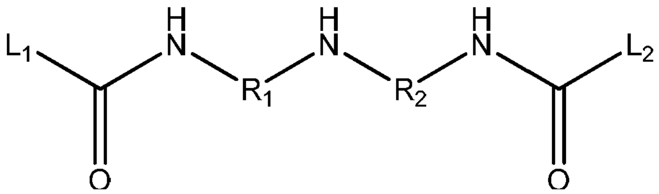

wherein

- $R_1$ and $R_2$ are aliphatic chains $(CH_2)_m$ and $(CH_2)_n$ wherein m and n are an integer in the range of 3 to 15, preferably 3 to 8, and
- $L_1$ and $L_2$ are blocking groups, preferably selected from caprolactam, phenol, oxime, triazole and malonic esters.

Polymerization of $AB_2$ monomers can be performed directly on an (activated) surface, for instance a titanium or titanium-alloy surface. Alternatively, in analogy to what is disclosed in WO2016/043584, it may comprise the 'prepolymerization' of $AB_2$ monomers to a low molecular weight polyurea, which low molecular weight polyurea is then contacted with the surface. The surface is preferably provided with a coupling agent. The surface may be activated prior to contacting with coupling agent.

Accordingly, in one embodiment the step of providing a substrate that is coated with an amine functionalized hyperbranched polyurea comprises

a) providing a surface, optionally comprising reactive hydroxyl groups, and grafting onto said (hydroxylated) surface a coupling agent;

b) polycondensation of $AB_2$ monomers comprising a secondary amine as A-group and blocked isocyanates as B-groups to obtain a low molecular weight polyurea of at least 1500 Da; and

c) contacting said low molecular weight polyurea with the surface grafted with a coupling agent to covalently anchor the polyurea, and continuing polycondensation by heating, optionally in the presence of $AB_2$ monomers, to obtain a hyperbranched polyurea coating.

Various types of coupling agents can be used, including 2-oxo-N(3-triethoxysilyl)propyl)azepane-1-carboxamide previously disclosed in WO2016/043584.

However, very good results in terms of coating strength were obtained with the development of a novel, dopamine-based coupling agent of the formula

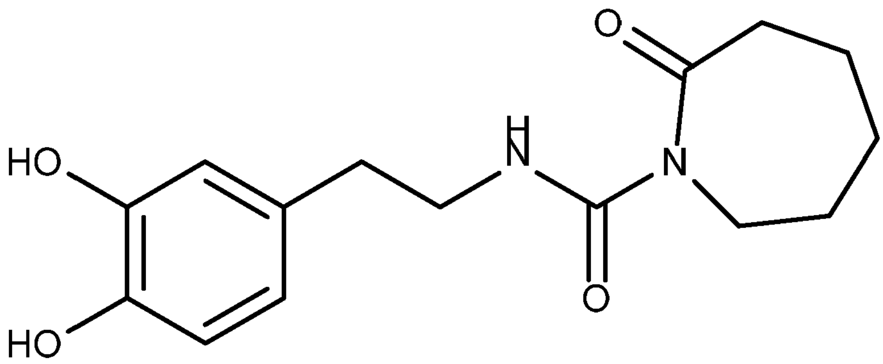

*N*-(3,4-dihydroxyphenethyl)-2-oxoazepane-1-carboxamide

This coupling agent is readily synthesized by reacting dopamine or a salt thereof, preferably dopamine.HCl, with carbonyl biscaprolactam (CBC). Also disclosed is a method for providing N-(3,4-dihydroxyphenethyl)-2-oxooazepane-1-carboxamide (CaBiDA), comprising reacting dopamine or a salt thereof with CBC in a suitable solvent in the presence of a base. Preferred solvents are polar solvents having a boiling temperature of 80°C or higher, preferably 100°C or higher, and show a good dopamine salt solubility. For example, the solvent comprises at least 10 w% of the dopamine salt. A particularly preferred solvent is DMF. Preferred bases include trimethylamine. In a specific aspect, the method comprises reacting stoichiometric amounts of CBC, dopamine hydrochloric acid salt and trimethylamine in DMF at about 80 °C in a nitrogen atmosphere.

Disclosed herein is the compound *N*-(3,4-dihydroxyphenethyl)-2-oxooazepane-1-carboxamide, herein referred to as CaBiDA.

Further disclosed is the use of CaBiDA as coupling agent, preferably as coupling agent in the interphase region between (i) an inorganic solid substrate, such as a glass, metal, or mineral substrate, and (ii) an organic substrate, such as an organic polymer, coating, or adhesive.

As is exemplified herein below, the novel coupling agent was found to exhibit an increased stability under wet conditions as compared to 2-oxoN(3-triethoxysilyl)propyl)azepane-1-carboxamide previously disclosed in WO2016/043584.

Herein disclosed is the use of CaBiDA as coupling agent in the interphase region between (i) a solid substrate, such as a glass substrate, a (medical grade) polymer substrate, a metal substrate, or a mineral substrate, and (ii) an antibacterial coating, preferably an antibacterial coating comprising non-alkylated PEI as disclosed herein above.

Also provided herein is a method of providing a solid substrate with a coating material, preferably a polymer coating, more preferably an antimicrobial or bactericidal coating, comprising contacting a surface of the substrate with CaBiDA coupling agent to mediate binding between the solid surface and the coating.

The substrate to be coated can be any type of substrate, article, object or material. In modern medicine frequent use is made of exogenous articles in such a way that they come into medium - or long-term contact with tissue or body fluids. Examples are implants, such as pacemakers, stents and prostheses, and also suture materials, drainage hoses and catheters. Such articles may consist, inter alia, of metals, ceramics and/or polymers.

In one embodiment, the substrate is a medical grade material, preferably selected from the group consisting of medical grade polyethylene, polydimethylsiloxane elastomer (PDMS), polyurethane, and polyvinylchloride (PVC).

In another embodiment, the substrate is a metal which is biocompatible with the mammalian body, preferably selected from the group consisting of stainless steel alloys, titanium, titanium-alloy, tantalum and tantalum-alloy.

Herein disclosed is a method for providing a solid substrate or at least part of a surface thereof with an antibacterial coating, comprising contacting the substrate with the CaBiDA coupling agent to obtain a surface that is grafted with coupling agent, followed by covalently anchoring an antibacterial coating, for example a hyperbranched polymer, which may be functionalized with a polyamine, such a non-quaternized or a quaternized PEI.

The coupling agent can simply be contacted (e.g. by immersion) with at least part of the surface with a solution of coupling agent, followed by continued reaction at elevated temperature. The surface may be activated or not. For example, an activated surface is immersed for 5-15 minutes in an alcoholic (e.g. MeOH) solution of coupling agent, and thereafter maintained at about 100-120°C for at least 1 hour, preferably at least 2 hours, to allow for adhesion of coupling agent. Unreacted coupling agent is preferably removed by washing, e.g. in ethanol, under sonication.

The CaBiDA coupling agent is treated with an oxidizing agent, such as $NaIO_4$, at room temperature, improving the adhesion. By increasing the pH to a value above 8, an additional effect on adhesion can be obtained. Optionally, a diamine such as 1,6-hexamethylene diamine can be added in addition to the previous mentioned methods.

The surface provided with CaBiDA is then reacted with a polymer that is readily functionalized to a specialty

coating by introducing of a wide variety of (bio-active) functionalities, such as biocides. Of particular interest are hyperbranched polymers including hyperbranched polyurea, discussed herein above. For example, to obtain a coating having antibacterial properties, an antibacterial functionality is coupled to the hyperbranched coating. This may include immobilizing onto the hyperbranched (polyurea) coating a hydrophobic, optionally N-alkylated, polyamines, such as polyethylenimine (PEI) having antibacterial properties. The antibacterial PEI may be obtained via the conventional route involving N-alkylation with linear, cyclic or branched C5-C15 alkyl chains, or aromatic compounds, such as benzyl. However, it is preferred that the above mentioned novel approach is used, which instead of N-alkylation comprises the use of an aqueous salt solution capable of reducing repulsive forces between positively charged nitrogen atoms of said amines in order to enhance the degree of PEI protonation, and therewith the biocidal activity.

Also provided herein is a substrate coated with an antibacterial coating obtainable by a method of the invention, which is defined by the appended claims. Also disclosed herein is a method that may involve the use of a shielding composition, the CaBiDA coupling agent and/or the additional contacting with a proteinaceous substance as herein disclosed.

The solid substrate is a medical device or implant, preferably selected from the group consisting of a catheter, a prosthesis, an orthopedic implant and a cardiovascular implant. Exemplary medical devices include: (1) extracorporeal devices for use in surgery such as blood oxygenators, blood pumps, blood sensors, tubing used to carry blood and the like which contact blood which is then returned to the patient; (2) prostheses implanted in a human or animal body such as vascular grafts, stents, pacemaker leads, heart valves, and the like that are implanted in blood vessels or in the heart; (3) devices for temporary intravascular use such as catheters, guide wires, and the like which are placed into blood vessels or the heart for purposes of monitoring or repair.

Encompassed are continuous coatings covering the entire substrate but also discontinuous local coatings or combinations of local coatings and continuous top coatings. A substrate disclosed herein is characterized by a strongly adhered antibacterial coating which displays a high mechanical strength.

The substrate is for example a medical device or implant, for instance a catheter or a prosthesis. In one embodiment, the implant is an orthopedic implant or a cardiovascular implant, for example selected from the group consisting of cardiac valves, alloplastic vessel wall supports, and total artificial heart implants. In another embodiment, the implant is selected from the group consisting of ear tubes, endotracheal tubes, ventilation tubes, cochlear implants and bone anchored hearing devices.

Notably, an antibacterial coating obtained by treating non-quaternized PEI with a polarizable salt has a more hydrophilic character as compared to the traditional *N*-alkylated quaternized PEI coatings. Since hydrophilic coatings are less toxic than hydrophobic coatings and may also promote cell growth, this property is particularly advantageous for coated implants or any other substrate or device to be used exposed to cells or tissue, including *in vitro* and *in vivo* applications.

An antimicrobial coating obtained according to the present invention may be provided with one or more further components that are of benefit for *in vitro* and *in vivo* applications, in particular for applications wherein the coating will be exposed to (mammalian) cells or tissue. For example, the coating can be provided with a component that reduces the adverse effect of the charge density on surrounding cells or tissue, without sacrificing the bactericidal effect.

In one embodiment, peptides or proteins are suitably used to promote or support tissue homeostasis and/or growth in the area in contact or surrounding a bactericidal coating or coated substrate, e.g. a biomedical implant. Therefore, also disclosed is that the step of treating the polyamine functionalized polymer with an aqueous salt solution to obtain an antimicrobial coating is followed by contacting the bactericidal coating with one or more proteinaceous substances such that "protective" proteinaceous substances become absorbed to the coating.

Thus, herein disclosed is a strongly adhered antimicrobial coating which displays a high mechanical strength and has a good compatibility with (mammalian) cells or tissue.

Preferred proteinaceous substances include peptides and proteins that are non-toxic, for example one or more naturally occurring mammalian, preferably human, protein(s) or fragment(s) thereof. Also encompassed are synthetic peptides or proteins. The use of one or more purified proteins is preferred.

In a specific aspect, the proteinaceous substance is a proline-rich protein, for example a member of the proline-rich proteins (PRPs) family of salivary proteins produced by the parotid and submandibular glands and constitute nearly 70% of the total protein of human saliva. Basic and glycosylated PRPs are encoded by four genes, PRB1-PRB4, and acidic PRPs by two genes, PRH1 and PRH2. They are synthesized as precursor proteins (~150 amino acids), many of which are cleaved before secretion to generate more than 20 PRPs in saliva. It has been shown in the art that the biocidal efficacy of a hyperbranched coating functionalized with N-alkylated PEI was not reduced in the presence of saliva, which contains a large amount of proteins (Dong et al. Langmuir, 2019, 35, 43, p. 14108-14116). However, nothing was mentioned or suggested how this would affect cell or tissue homeostasis.

In another specific aspect, one or more mammalian serum proteins is used, including fibronectin, alpha-1-antitrypsin, alpha-2-macroglobulin, ceruloplasmin, transferrin and serum albumin. For example, the coating is contacted with human or bovine serum albumin

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

The invention is exemplified by the following non-limiting examples.

LEGEND TO THE FIGURES

Figure 1: $^1H$ NMR spectrum of the dopamine-based coupling agent *N*-(3,4-dihydroxyphenethyl)-2-oxooazepane-1-carboxamide (CaBiDA).

EXPERIMENTAL SECTION

**Example 1 : Synthesis of coupling agent CaBiDA.**

Stoichiometric amounts of carbonyl biscaprolactam (CBC; 1.419 g, 5.631 mmol), dopamine hydrochloric acid salt (1.068 g, 5.631 mmol), trimethylamine (0.784 mL, 5.631 mmol) and 10 mL DMF were added to round bottom flask which was equipped with a reflux condenser, and stirred at 80 °C for 24 h in a nitrogen atmosphere. After cooling down the solution the coupling agent was precipitated by adding 50 mL of an aqueous $CaCl_2$ solution (5wt%) and then dissolved in 10 mL $CHCl_3$. After removal of the aqueous layer, the organic layer was washed two times with 50 mL of an aqueous solution of $CaCl_2$ (5wt%) and once with 50 mL of a saturated brine solution. After removal of the aqueous layer, the organic layer was dried on anhydrous magnesium sulfate ($MgSO_4$). After drying, $MgSO_4$ was filtered off and the solvent was completely removed in a rotavapor under reduced pressure (yield 97%). The $^1H$ NMR spectrum shown in Figure 1 confirmed the structure of CaBiDA.

A reaction scheme is given in scheme 1.

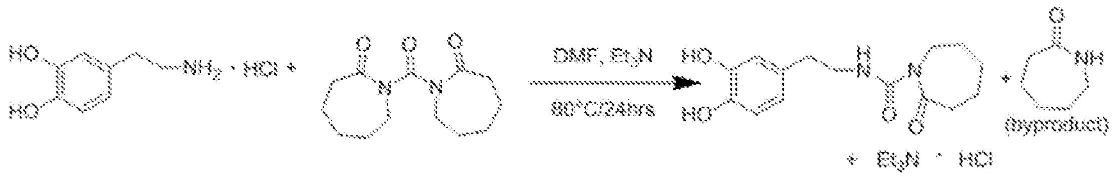


Scheme 1: Preparation of dopamine-based coupling agent.

**Example 2 : Synthesis of $AB_2$ molecules and hyperbranched polyurea polymer (HBP)**

Carbonyl biscaprolactam (CBC, 5.805 g, 23 mmol), bishexamethylene triamine (2.495 g, 11.5 mmol) and 10 mL xylene were added into a roundbottom flask equipped with a reflux condenser and stirred heated at 80 °C for 8 h in a nitrogen atmosphere to obtain the $AB_2$ monomer. Next, the temperature was raised to 145 °C for 1 h reaction to induce prepolymerization, yielding a number average molecular weight of 1,500Da.

The solution was cooled down to room temperature, 5 mL of xylene was added and extracted three times with an aqueous $CaCl_2$ solution (5 wt%), and once with a saturation brine solution. After removal of the aqueous layer, the organic layer was dried on anhydrous magnesium sulfate ($MgSO_4$). After drying $MgSO_4$ was filtered off and the solvent was completely removed in a rotavapor under reduced pressure, yielding a waxy solid. In scheme 2 an overview of the reactions is depicted.

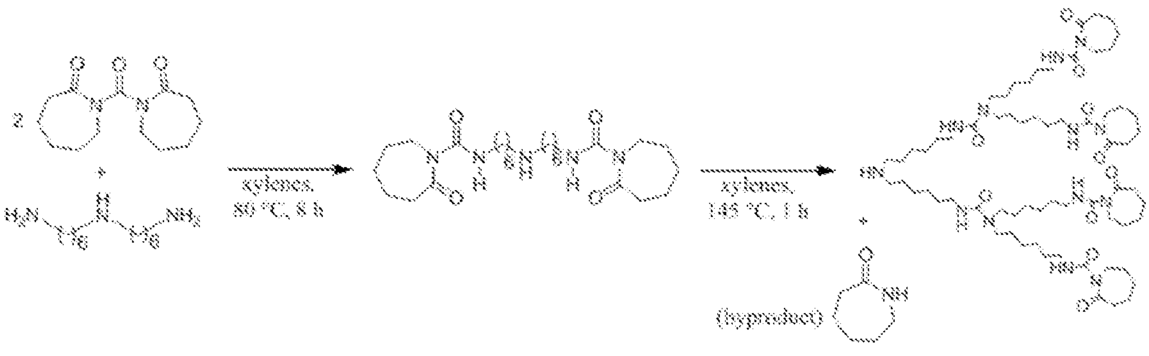


Scheme 2: Schematic representation of the preparation of $AB_2$ monomer and hyperbranched polymer (HBP)

**Example 3: Pre-treatment of a titanium substrate**

Titanium samples (Ti, 10 mm×10 mm×1 mm) were cleaned by dispersing them in a sonication bath in n-hexane for 10 min, followed by 30 min ultrasonic immersion in 40 mL of 10 M HCl solution, subsequently immersed in 40 mL milli-Q water and sonicated for 30 min. Then, the Ti samples were rinsed with acetone and dried at room temperature (RT). Next, the Ti samples were oxidized in a mixture of 25% $NH_4OH$, 30% $H_2O_2$ and $H_2O$ (1:1:5 v/v) at 80 °C for 20 min. After this treatment. Ti samples were washed with milli-Q water, followed by washing with acetone and dried at RT.

**Example 4: Application of coupling agent**

A) The pre-treated Ti substrates (Example 3) were immersed into a solution of the coupling agent (Example 1) in ethanol (3 wt%) for 10 min, and then heated at 110 °C for 2h in a vacuum oven. The unreacted coupling agent was removed by sonicating the samples with ethanol for 20 min at RT and dried.

B) Alternatively, a pre-treated Ti substrate (Example 3) was immersed into a PBS solution comprising CaBiDA and $NaIO_4$ (molar ratio 2:1) at a pH of 7.0 and incubated at 37 °C overnight, and then washed ultrasonically with ethanol for 10 min and dried.

**Example 5: Immobilization of hyperbranched polymer**

A solution of HBP (Example 2; 5 wt% in ethanol) was spin-coated (2000 rpm, 60 s) on the Ti pieces covered with the coupling agent (Example 4A). Then the samples were heated for 2 h at 145 °C in a nitrogen atmosphere. Non-anchored polymers were removed by an extraction in DMF for 2 h at 115 °C. Next, the coated Ti pieces were sonicated in methanol for 20 min at RT and dried.

**Example 6: Functionalization with polyethyleneimine (PEI)**

100 μL of PEI solution (20 wt% in methanol) was dropped on the coated titanium samples (example 5) and spin coated (2000 rpm, 60s). The anchoring reactions were carried out at 125 °C for 3 hours in a nitrogen atmosphere. Unreacted PEI was removed by sonication with methanol for 20 min at RT and dried.

**Example 7: Enhancing the charge density of PEI**

The PEI-coated samples (example 6) were immersed in several aqueous solutions comprising different types of salts for 2-20 h at room temperature (see Table 1), followed by 3 times washing with pure water and sonicating once for 10 min in pure water. The resulting charge density was measured according to the fluorescein method (example 8),

and expressed as percentage increase relative to the charge density prior to immersion. Furthermore, the antibacterial properties of a number of samples was tested in a Petri film assay with *S. epidermidis* as bacterial strain (example 9).

Table 1. Charge densities and antibacterial properties of PEI-coated titanium substrates after exposure to aqueous solutions comprising different types and concentrations of salts.

| Salt | Incubation Time (h) | Polarizability According to * | Charge density increase | Antibacteri al |
|---|---|---|---|---|
| None | | | - | No |
| NaCl (10 mM) | 20 | 3.359 | 0% | No |
| NaI (1 mM) | 20 | 7.443 | 39% | Yes |
| NaI (10 mM) | 2 | 7.443 | ND | Yes |
| NaI (10 mM) | 8 | 7.443 | ND | Yes |
| NaI (10 mM) | 20 | 7.443 | 81% | Yes |
| NaI (25 mM) | 20 | 7.443 | 105% | Yes |
| NaI (40 mM) | 20 | 7.443 | 134% | Yes |
| NaI (100 mM) | 20 | 7.443 | 135% | Yes |
| $Mg(NO_3)_2$ (10 mM) | 20 | 7.475 | 26% | Yes |
| $Zn(NO3)_2$ (1 mM) | 2 | ND | ND | Yes |
| NaNOs (1 M) | 2 | 4.313 | 63% | Yes |
| # Li et al. (J. Phys. Chem. B, 2017, 121, 6416-6424) who determined salt polarizability values at 37°C presented as $\alpha_{37}$ in the last column of Table 1.<br>ND: not determined | | | | |

**Example 8: Two-step *N*-alkylation of PEI (Comparative example)**

In a round bottom flask provided with a reflux condenser, coatings comprising tethered PEI sample (example 6) were N-alkylated (quaternized) by immersion in 20 mL $C_6H_{13}Br$ at 90 °C for 4 h. Next, 1.07 g of a proton sponge (1,8-bis(dimethylamino)naphthalene) in 25 mL of *tert*-amyl alcohol was added. The reaction was continued for 3 h. The coated samples were rinsed with methanol and subsequently sonicated for 20 min at RT. The obtained coatings were dried and placed into round bottom flask in a nitrogen atmosphere for a second alkylation step. In a round bottom flask was provided with a reflux condenser and the samples were immersed in 20 mL $CH_3I$ at 42 °C for 24 h. The coated samples were rinsed with methanol and subsequently sonicated for 20 min at RT, then dried and stored in bottle in a nitrogen atmosphere. The increase of the charge density due to both alkylation steps was 109%. The antibacterial properties were determined as described in example, with *S. epidermidis* bacteria and all were killed.

**Example 9: Charge density measurements**

Samples were immersed at RT in 15 mL of a 1wt% fluorescein (disodium salt) solution in demineralized water for 10 min, washed four times with 50 mL water, followed by sonication in 50 mL water for 5 min at RT to remove any dye not complexed with cationic species, and dried with an air flow to remove residue water. Next, the samples were placed in 10 mL of a 0.1 wt% cetyltrimethylammonium chloride solution in demineralized water and sonicated for 10 min at RT to desorb complexed fluorescein dye. Subsequently, 10 v/v% of 100 mM phosphate buffer, pH 8, was added to a total volume of 11 mL and UV/VIS measurements (Spectra max M2 UV/VIS spectrophotometer) carried out at 501 nm. The charge density was calculated according to the method as described by Steven Roest, Henny C. van der Mei, Ton J.A. Loontjens, Henk J. Busscher, in Applied Surface Science 356 (2015) 325-332. The results are indicated in Table 1.

**Example 10: Antibacterial evaluation by the Petrifilm test**

*S. epidermidis* ATCC 12228 was first streaked on a blood agar plate from a frozen stock solution (7 v/v% DMSO) and grown overnight at 37 °C on blood agar. One colony was inoculated in 10 mL tryptone soya broth (TSB, Oxoid, Basingstoke, UK) and incubated at 37 °C for 24 h. This culture was used to inoculate a main culture of 200 mL TSB, which was incubated for 16 h at 37 °C. Bacteria were harvested by centrifugation for 5 min at 5000 g and 10 °C and subsequently washed two times with 10 mM potassium-phosphate buffer, pH 7.0.

The ability of the coatings to kill adhering staphylococci was evaluated by the Petrifilm assay. The Petrifilm assay employed is based on culturing of organisms that survive contact with the coatings under nutrient-rich conditions. The Petrifilm Aerobic Count plate (3M Microbiology, St. Paul, MN, USA) consists of two films: a bottom film containing standard nutrients, a cold-water gelling agent and an indicator dye that facilitates colony counting and a top film enclosing the sample within the system. The bottom film containing the gelling-agent was first swelled with 1 mL sterile demineralized water for 40 min and transferred to the transparent top film before usage. Next, 10 $\mu$ L bacterial suspensions with the different concentrations were placed on coated slides (1 cm $\times$ 1 cm). After closure of the Petrifilm system with a slide in between, the staphylococcal suspension spread over the entire surface area of the samples, enabling calculation of the bacterial challenge per cm2 from the dimensions of the samples and the bacterial concentration in suspension. Petrifilms were incubated at 37 °C for 48 h after which the numbers of CFU were counted. As a control, 10 $\mu$ L of the bacterial suspension was inoculated on Petrifilm without a sample in between.

**Example 11: Covalently attached Antibacterial coating on PDMS**

PDMS pieces (2 $\times$ 2.5 $cm^2$) were placed in an air plasma equipment (Femto system from Diener-electronic, Germany) at 100 W, for 1-2 min (at $1.7\times10^{-1}$mbar air). The obtained hydrophilic PDMS pieces were immersed in the 3 v/v% solution of CaBiDA in absolute ethanol for 10 min at RT and then placed in vacuum oven and heated at 110°C for 2 h under vacuum. The unreacted coupling agent was removed by washing PDMS pieces in ethanol for 20 min in sonic bath at RT and dried under vacuum and stored under nitrogen.

PDMS slides were submerged in a solution of hyperbranched polymer (5 wt% in ethanol) and subsequently spin coated (2000 rpm, 60 s). On heating at 145 °C for 2 h fixation on the coupling agent and a continued polymerization of the hyperbranched polymer on the surface was carried out under a nitrogen flow. Non-anchored polymers were removed by an extraction in 200 mL DMF at 115 °C for 2h. Next PDMS pieces were sonicated in absolute ethanol for 20 min at RT and dried and stored under nitrogen.

A solution of polyethyleneimine (PEI) in water (50 wt%) was freeze dried overnight ($M_w$=750 kDa) and the residue was dissolved in methanol in 20 wt% concentrations. 200 $\mu$L of the PEI solution was dropped on the sample covered with the hyperbranched polymer and spin coated (2000 rpm, 60 s). The anchoring reactions were carried out on aluminium plate at 125 °C for 3 under nitrogen. Unreacted PEI was removed with methanol using ultrasonic bath for 45 min at RT and dried under nitrogen.

The PEI-coated PDMS piece were immersed at RT in 10 mmol/L NaI solution for 2, 8 and 20h respectively, followed by 3 times washing with pure water and sonicating once for 10 min in pure water. The resulting charge density was measured according to the fluorescein method and expressed as percentage increase relative to the charge density prior to immersion.

The charge density increase of the sample after 20h immersion was 30%. The antibacterial properties of the samples after 2 and 8h immersion were tested with *Staphylococcus Epidermidis* as described in Example 10. No surviving bacteria were observed.

**Example 12: CaBiDA shows improved adhesion.**

The CaBiDA coupling agent was applied on a titanium substrate using the $NaIO_4$ route as described in example 4B. The siloxane coupling agent 2-oxoN(3-triethoxysilyl)propyl)azepane-1-carboxamide (CaBiTES) previously disclosed in WO2016/043584 was used as comparative example. The samples were immersed in water for 4 days at room temperature. The contact angles measured before and after immersion are presented in Table 2.

Table 2. Contact angles of titanium samples covered with CaBiDA or CaBiTES.

| Coupling agent | Before immersion | After 4 days immersion |
|---|---|---|
| CaBiDA | 38.8 | 37.8 |
| CaBiTES | 71.0 | 46.9 |

The contact angle of CaBiDA remained the same, while the contact angle of CaBiTES went down from 71.0 to 46.9 degrees, indicating a reduced stability of CaBiTES under wet conditions.

**References**

[1] Bactericidal Properties of Flat Surfaces and Nanoparticles Derivatized with Alkylated Polyethylenimines, Jian Lin, Shuyi Qiu, Kim Lewis, and Alexander M. Klibanov, Biotechnol. Prog., 2002, Vol. 18, No. 5,1082-1086.
[2] PLOS, Unusual Salt and pH Induced Changes in Polyethylenimine Solutions, Kimberly A. Curtis , Danielle Miller , Paul Millard, Saswati Basu, Ferenc Horkay , Preethi L Chandran, September 29, 2016, 1-20.
[3] A Shape-Adaptive, Antibacterial-Coating of Immobilized Quaternary-Ammonium Compounds Tethered on Hyperbranched Polyurea and its Mechanism of ActionLia A. T. W. Asri, Mihaela Crismaru , Steven Roest, Yun Chen , Oleksii Ivashenko , Petra Rudolf, Joerg C. Tiller , Henny C. van der Mei, Ton J. A. Loontjens , and Henk J. Busscher, Adv. Funct. Mater. 2014, 24, 346-355.
[4] Woongmo Sung, Zaure Avazbaeva, and Doseok Kim, Salt Promotes Protonation of Amine Groups at Air/Water Interface, J. Phys. Chem. Lett. 2017, 8, 3601-3606.
[5] Antimicrobial Surfaces, Joerg C. Tiller, Advances in Polymer Science 2010, 240(1):193-217.
[6] Permanent, non-leaching antibacterial surfaces-2: How high density cationic surfaces kill bacterial cells, Hironobu Murata, Richard R. Koepsel, Krzysztof Matyjaszewskic, Alan J. Russell, Biomaterials 28 (2007) 4870-4879.
[7] Macromol Biosci. 2012 ; 12(9): 1279-1289, Poly(ethylene imine)s as antimicrobial agents with selective activity, Katherine Gibney, Iva Sovadinova, Analette I. Lopez, Michael Urban, Zachary, Ridgway, Gregory A. Caputo, and Kenichi Kuroda.
[8] Charge properties and bacterial contact-killing of hyperbranched polyurea-polyethyleneimine coatings with various degrees of alkylation Steven Roest, Henny C. van der Mei, Ton J.A. Loontjens, Henk J. Busscher, Applied Surface Science 356 (2015) 325-332.


**Claims**

1. A method for providing a substrate with an antimicrobial coating, comprising providing a substrate that is covalently coated with a polyamine-functionalized polymer, wherein said polyamine is non-quaternized and non-alkylated polyethyleneimine (PEI), and contacting said polyamine-functionalized polymer with an aqueous salt solution comprising at least one salt having a polarizability $\alpha_{37}$ of at least 4 $Å^3$ determined at 37°C wherein

$$\alpha_{salt} = \frac{3R}{4\pi N}$$

wherein R is molar refractivity, N is Avogadro's constant and $\alpha_{salt}$ denotes the sum of polarizabilities of the solvated ions in cgs units, and wherein said aqueous salt solution comprises an ammonium salt, an alkaline metal salt or earth alkaline metal salt of an anion selected from the group consisting of Br-, I-, $ClO_4^-$, $SO_4^{2-}$, $NO_3^-$ and $PO_4^{3-}$, or $Zn(NO_3)_2$, or $Al(NO_3)_3$.

2. Method according to claim 1, wherein said aqueous salt solution comprises at least one salt having a polarizability $\alpha$37 of at least 4.5 $Å^3$.

3. Method according to claim 1 or 2, wherein said aqueous salt solution comprises one or more of NaI, KI, NaBr, KBr, $NaClO_4$, $KClO_4$, $Na_2SO_4$, $K_2SO_4$, $Na_3PO_4$, $K_3PO_4$, $Mg(NO_3)_2$, $Ca(NO_3)_2$, $Zn(NO_3)_2$, $NaNO_3$, $(NH_4)_2SO_4$, $NH_4NO_3$, $MgSO_4$, $NH_4I$, $CaSO_4$, and $Al(NO_3)_3$.

4. Method according to any one of claims 1-3,wherein said aqueous salt solution comprises NaI.

5. Method according to any one of claims 1-4, followed by contacting said polyamine-functionalized polymer with one or more proteinaceous substances.

6. Method according to any one of claims 1-5, wherein the substrate is a medical grade material, preferably selected from the group consisting of medical grade polyethylene, polydimethylsiloxane elastomer (PDMS), polyurethane,

and polyvinylchloride (PVC).

**7.** Method according to any one of claims 1-5, wherein the substrate is a material which is biocompatible with the mammalian body, preferably selected from the group consisting of ceramics, stainless steel alloys, titanium, titanium-alloy, tantalum and tantalum-alloy.

**8.** Method according to any one of the preceding claims, wherein the polymer coating is covalently associated with at least part of the outer surface of the substrate.

**9.** Method according to any one of the preceding claims, wherein the polymer coating comprises a polyurea coating, preferably a hyperbranched polyurea coating.

**10.** Method according to claim 9, wherein said hyperbranched polyurea coating is obtained by

a) providing a surface, optionally comprising reactive hydroxyl groups, and covalently grafting onto said (hydroxylated) surface a coupling agent;
b) polycondensation of $AB_2$ monomers comprising a secondary amine as A-group and blocked isocyanates as B-groups to obtain a number average molecular weight polyurea of at least 1500 Da; and
c) contacting said low molecular weight polyurea with the surface grafted with a coupling agent to covalently anchor the polyurea, and continuing polycondensation by heating, optionally in the presence of $AB_2$ monomers, to obtain a hyperbranched polyurea coating.

**11.** Method according to claim 10, wherein said coupling agent is

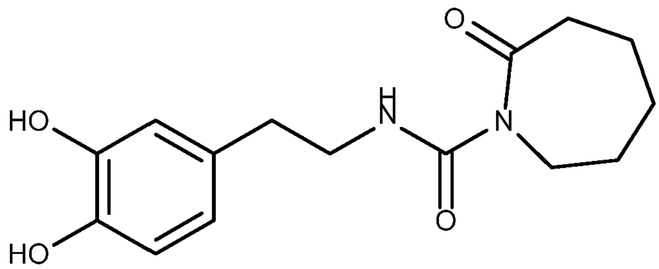

*N*-(3,4-dihydroxyphenethyl)-2-oxoazepane-1-carboxamide

**12.** A coated substrate obtainable by a method according to any one of claims 1-11.

## Patentansprüche

**1.** Verfahren zum Bereitstellen eines Substrats mit einer antimikrobiellen Beschichtung, umfassend Bereitstellen eines Substrats, das kovalent mit einem polyaminfunktionalisierten Polymer beschichtet ist, wobei das Polyamin nicht-quaternisiertes und nicht-alkyliertes Polyethylenimin (PEI) ist, und Inkontaktbringen des polyaminfunktionalisierten Polymers mit einer wässrigen Salzlösung, umfassend mindestens ein Salz mit einer Polarisierbarkeit $\alpha_{37}$ von mindestens 4 Å$^3$, bestimmt bei 37 °C, wobei

$$\alpha_{salt} = \frac{3R}{4\pi N}$$

wobei R molare Refraktivität ist, N Avogadro-Konstante ist und $\alpha_{Salz}$ die Summe von Polarisierbarkeiten der solvatisierten Ionen in cgs-Einheiten bezeichnet, und wobei die wässrige Salzlösung ein Ammoniumsalz, ein Alkalimetallsalz oder Erdalkalimetallsalz eines Anions, ausgewählt aus der Gruppe bestehend aus Br-, I-, $ClO_4$-, $SO_4^{2-}$, $NO_3$- und $PO_4^{3-}$, oder $Zn(NO_3)_2$ oder $Al(NO_3)_3$ umfasst.

**2.** Verfahren nach Anspruch 1, wobei die wässrige Salzlösung mindestens ein Salz mit einer Polarisierbarkeit $\alpha_{37}$ von mindestens 4,5 Å$^3$ umfasst.

3. Verfahren nach Anspruch 1 oder 2, wobei die wässrige Salzlösung eines oder mehrere von NaI, KI, NaBr, KBr, $NaClO_4$, $KClO_4$, $Na_2SO_4$, $K_2SO_4$, $Na_3PO_4$, $K_3PO_4$, $Mg(NO_3)_2$, $Ca(NO_3)_2$, $Zn(NO_3)_2$, $NaNO_3$, $(NH_4)_2SO_4$, $NH_4NO_3$, $MgSO_4$, $NH_4I$, $CaSO_4$, und $Al(NO_3)_3$ umfasst.

4. Verfahren nach einem der Ansprüche 1 - 3, wobei die wässrige Salzlösung NaI umfasst.

5. Verfahren nach einem der Ansprüche 1 - 4, gefolgt von Inkontaktbringen des polyaminfunktionalisierten Polymers mit einer oder mehreren proteinhaltigen Substanzen.

6. Verfahren nach einem der Ansprüche 1 - 5, wobei das Substrat ein Material medizinischer Qualität ist, vorzugsweise ausgewählt aus der Gruppe bestehend aus Polyethylen, Polydimethylsiloxanelastomer (PDMS), Polyurethan, und Polyvinylchlorid (PVC) medizinischer Qualität.

7. Verfahren nach einem der Ansprüche 1 - 5, wobei das Substrat ein Material ist, das mit dem Säugetierkörper biokompatibel ist, vorzugsweise ausgewählt aus der Gruppe bestehend aus Keramik, Edelstahllegierungen, Titan, Titanlegierung, Tantal und Tantallegierung.

8. Verfahren nach einem der vorhergehenden Ansprüche, wobei die Polymerbeschichtung kovalent mit mindestens einem Teil der äußeren Oberfläche des Substrats verbunden ist.

9. Verfahren nach einem der vorhergehenden Ansprüche, wobei die Polymerbeschichtung eine Polyharnstoffbeschichtung, vorzugsweise eine hoch verzweigte Polyharnstoffbeschichtung umfasst.

10. Verfahren nach Anspruch 9, wobei die hoch verzweigte Polyharnstoffbeschichtung erhalten wird durch

    a) Bereitstellen einer Oberfläche, optional umfassend reaktive Hydroxylgruppen, und kovalentes Pfropfen eines Kupplungsmittels auf die (hydroxylierte) Oberfläche;
    b) Polykondensation von $AB_2$-Monomeren, umfassend ein sekundäres Amin als A-Gruppe und blockierte Isocyanate als B-Gruppen, um einen Polyharnstoff mit einer zahlenmittleren Molmasse von mindestens 1500 Da zu erhalten; und
    c) Inkontaktbringen des Polyharnstoffs mit niedriger Molmasse mit der Oberfläche, gepfropft mit einem Kupplungsmittel, um den Polyharnstoff kovalent zu verankern, und Fortsetzen der Polykondensation durch Erhitzen, optional in Gegenwart von $AB_2$-Monomeren, um eine hoch verzweigte Polyharnstoffbeschichtung zu erhalten.

11. Verfahren nach Anspruch 10, wobei das Kupplungsmittel

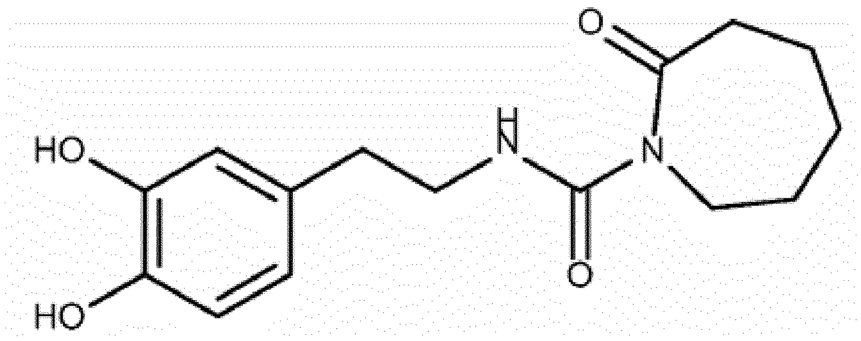

N-(3,4-Dihydroxyphenethyl)-2-oxoazepan-1-carboxamid

ist.

12. Beschichtetes Substrat, erhältlich durch ein Verfahren nach einem der Ansprüche 1 - 11.

**Revendications**

1. Un procédé pour doter un substrat d’un revêtement antimicrobien, comprenant la fourniture d’un substrat qui est

revêtu de manière covalente d'un polymère fonctionnalisé par une polyamine, dans lequel ladite polyamine est une polyéthylèneimine (PEI) non quaternisée et non alkylée, et la mise en contact dudit polymère fonctionnalisé par une polyamine avec une solution saline comprenant au moins un sel ayant une polarisabilité $\underline{\alpha}_{37}$ d'au moins 4Å$^3$ déterminée à 37°C, dans laquelle

$$\alpha_{sel} = \frac{3R}{4\pi N}$$

dans laquelle R est la réfractivité molaire, N est la constante d'Avogadro et $\alpha_{saline}$ désigne la somme des polarisabilités des ions solvatés en unités CGS, et dans lequel ladite solution aqueuse saline comprend un sel d'ammonium, un sel de métal alcalin ou un sel de métal alcalino-terreux d'un anion choisi parmi le groupe constitué Br-, I-, ClO4-, $SO_4^{2-}$, $NO_3$- et $PO4^{3-}$ ou $Zn(NO_3)_2$, ou Al $(NO_3)_3$ .

**2.** Procédé selon la revendication 1, dans lequel ladite solution aqueuse saline comprend au moins un sel ayant une polarisabilité $\alpha_{37}$ d'au moins 4,5Å$^3$.

**3.** Procédé selon la revendication 1 ou 2, dans lequel ladite solution saline aqueuse comprend un ou plusieurs parmi NaI, KI, NaBr, KBr, $NaC1O_4$, $KClO_4$, $Na_2SO_4$, $K_2SO_4$, $Na_3PO_4$, $K_3PO_4$, $Mg(NO_3)_2$, $Ca(NO_3)_2$, $Zn(NO_3)_2$, $NaNO_3$, $(NH_4)_2S0_4$, $NH_4NO_3$, $MgSO_4$, $NH_4I$, $CaSO_4$ et $Al(NO_3)_3$.

**4.** Procédé selon l'une quelconque des revendications 1 à 3, dans lequel ladite solution saline aqueuse comprend du NaI.

**5.** Procédé selon l'une quelconque des revendications 1 à 4, suivi de la mise en contact dudit polymère fonctionnalisé par une polyamine avec une ou plusieurs substances protéiques.

**6.** Procédé selon l'une quelconque des revendications 1 à 5, dans lequel le substrat est un matériau de qualité médicale, de préférence choisi dans le groupe constitué du polyéthylène de qualité médicale, de l'élastomère polydiméthyl siloxane (PDMS), du polyuréthane et du chlorure de polyvinyle (PVC).

**7.** Procédé selon l'une quelconque des revendications 1 à 5, dans lequel le substrat est un matériau biocompatible avec le corps du mammifère, de préférence choisi dans le groupe constitué de la céramique, des alliages d'acier inoxydable, du titane, de l'alliage de titane, du tantale et de l'alliage de tantale.

**8.** Procédé selon l'une quelconque des revendications précédentes, dans lequel le revêtement polymère est associé de manière covalente à au moins une partie de la surface externe du substrat.

**9.** Procédé selon l'une quelconque des revendications précédentes, dans lequel le revêtement polymère comprend un revêtement de polyurée, de préférence un revêtement polyurée hyperbranché.

**10.** Procédé selon la revendication 9, dans lequel ledit revêtement de polyurée hyperbranchée est obtenu par

a) la fourniture d'une surface, comprenant éventuellement des groupes hydroxyles réactifs, et le greffage de manière covalente sur ladite surface (hydroxylée) d'un agent de couplage ;
b) la polycondensation de monomères $AB_2$ comprenant une amine secondaire comme groupe A et des isocyanates bloqués comme groupes B pour obtenir une polyurée de poids moléculaire moyen en nombre d'au moins 1500 Da; et
c) la mise en contact de ladite polyurée de faible poids moléculaire à surface greffée avec un agent de couplage pour ancrer de manière covalente la polyurée, et poursuivre la polycondensation par chauffage, éventuellement en présence de monomères $AB_2$, pour obtenir un revêtement de polyurée hyper-ramifiée.

**11.** Procédé selon la revendication 10, dans lequel ledit agent de couplage est

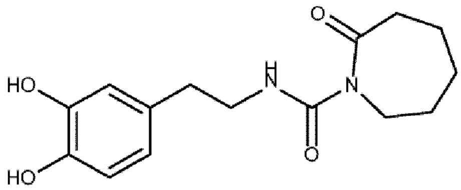

N-(3,4-dihydroxyphénéthyl)-2-oxoazépane-1-carboxamide

**12.** Substrat revêtu pouvant être obtenu par un procédé selon l'une quelconque des revendications 1 à 11.

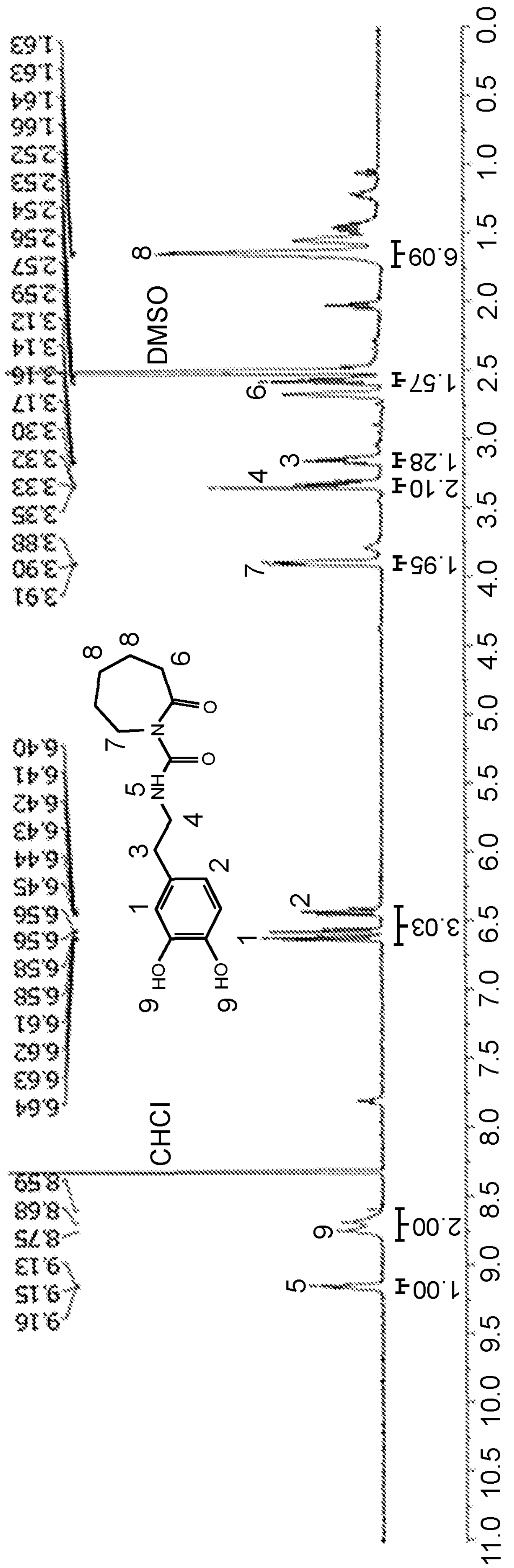


Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016043584 A
- US 20130110237 A
- US 20140112994 A
- WO 2008156636 A


### Non-patent literature cited in the description


- **BEHLAU et al.** *Biomaterials,* December 2011, vol. 32 (34), 8783-8796
- **PARK et al.** *Biotechnol. Prog.,* 2006, vol. 22, 584-589
- **LIN et al.** *Biotechnol. Prog.,* 2002, vol. 18, 1082-1086
- **ZHAO et al.** *Langmuir,* 2019, vol. 35 (17), 5779-5786
- **GIBNEY et al.** *Macromol. Biosc.,* vol. 12 (9), 1279-1289
- **ASRI et al.** *Adv. Funct. Mater.,* 2014, vol. 24, 346-355
- **YODOVIN-FARBER et al.** *J. of Nanomaterials,* vol. 2010, 1-11
- **ZALTSMAN et al.** *J. Appl. Biomater. Funct Mater,* 2016, vol. 14 (2), e205-e211
- **WONG et al.** *Biomaterials,* 2010, vol. 31, 4079-4087
- **LI et al.** *J. Phys. Chem. B,* 2017, vol. 121, 6416-6424
- *J. Phys. Chem. B,* 2017, vol. 121, 6416-6424
- **TILLER et al.** *PNAS,* 22 May 2001, vol. 98 (11), 5981-5985
- **ASRI et al.** *Adv. Funct. Mater,* 2014, vol. 24, 346-355
- **DONG et al.** *Langmuir,* 2019, vol. 35 (43), 14108-14116
- **STEVEN ROEST ; HENNY C. VAN DER MEI ; TON J.A. LOONTJENS ; HENK J. BUSSCHER.** *Applied Surface Science,* 2015, vol. 356, 325-332
- **JIAN LIN ; SHUYI QIU ; KIM LEWIS ; ALEXANDER M. KLIBANOV.** Bactericidal Properties of Flat Surfaces and Nanoparticles Derivatized with Alkylated Polyethylenimines. *Biotechnol. Prog.,* 2002, vol. 18 (5), 1082-1086
- **KIMBERLY A. CURTIS ; DANIELLE MILLER ; PAUL MILLARD ; SASWATI BASU ; FERENC HORKAY ; PREETHI L CHANDRAN.** Unusual Salt and pH Induced Changes in Polyethylenimine Solutions. *PLOS,* 29 September 2016, 1-20
- **LIA A. T. W. ASRI ; MIHAELA CRISMARU ; STEVEN ROEST ; YUN CHEN ; OLEKSII IVASHENKO ; PETRA RUDOLF ; JOERG C. TILLER ; HENNY C. VAN DER MEI ; TON J. A. LOONTJENS ; HENK J. BUSSCHER.** A Shape-Adaptive, Antibacterial-Coating of Immobilized Quaternary-Ammonium Compounds Tethered on Hyperbranched Polyurea and its Mechanism of Action. *Adv. Funct. Mater.,* 2014, vol. 24, 346-355
- **WOONGMO SUNG ; ZAURE AVAZBAEVA ; DO-SEOK KIM.** Salt Promotes Protonation of Amine Groups at Air/Water Interface. *J. Phys. Chem. Lett.,* 2017, vol. 8, 3601-3606
- **JOERG C. TILLER.** Antimicrobial Surfaces. *Advances in Polymer Science,* 2010, vol. 240 (1), 193-217
- **HIRONOBU MURATA ; RICHARD R. KOEPSEL ; KRZYSZTOF MATYJASZEWSKIC ; ALAN J. RUSSELL.** Permanent, non-leaching antibacterial surfaces-2: How high density cationic surfaces kill bacterial cells. *Biomaterials,* 2007, vol. 28, 4870-4879
- **KATHERINE GIBNEY ; IVA SOVADINOVA ; ANALETTE I. LOPEZ ; MICHAEL URBAN ; ZACHARY ; RIDGWAY ; GREGORY A. CAPUTO ; KENICHI KURODA.** Poly(ethylene imine)s as antimicrobial agents with selective activity. *Macromol Biosci.,* 2012, vol. 12 (9), 1279-1289
- **STEVEN ROEST ; HENNY C. VAN DER MEI ; TON J.A. LOONTJENS ; HENK J. BUSSCHER.** Charge properties and bacterial contact-killing of hyperbranched polyurea-polyethyleneimine coatings with various degrees of alkylation. *Applied Surface Science,* 2015, vol. 356, 325-332